Europäisches Patentamt

European Patent Office

Office européen des brevets

(11) Publication number: 0 073 277
B1

## EUROPEAN PATENT SPECIFICATION

(45) Date of publication of patent specification: 17.07.85

(21) Application number: 81303856.9

(22) Date of filing: 24.08.81

(51) Int. Cl.⁴: **C 07 C 85/24, C 07 C 87/50**

(54) Process for producing orthoalkylated aromatic amines.

(43) Date of publication of application:
09.03.83 Bulletin 83/10

(45) Publication of the grant of the patent:
17.07.85 Bulletin 85/29

(84) Designated Contracting States:
CH DE FR GB IT LI

(56) References cited:
GB-A-1 458 336
US-A-2 948 755
US-A-3 275 690
US-A-3 678 113
US-A-3 862 233
US-A-4 128 582

INDUSTRIAL AND ENGINEERING CHEMISTRY,
43 (7) (1951), pages 1579-1583

(73) Proprietor: MITSUI PETROCHEMICAL
INDUSTRIES, LTD.
2-5, Kasumigaseki 3-chome Chiyoda-ku
Tokyo 100 (JP)

(72) Inventor: Takahata, Kazunori
2-6, Misono 1-chome
Otake-shi Hiroshima-ken (JP)
Inventor: Taniguchi, Katsuo
2-4, Muronoki-cho 1-chome
Iwakuni-shi Yamaguchi-ken (JP)

(74) Representative: Perry, Robert Edward et al
GILL JENNINGS & EVERY 53-64 Chancery Lane
London WC2A 1HN (GB)

Courier Press, Leamington Spa, England.

# 0 073 277

## Description

The present invention relates to a process for selectively producing orthoalkylated aromatic amine at a high yield and, more specifically, it relates to a process for producing an orthoalkylated aromatic amine from an aromatic amine having at least one hydrogen atom at the ortho-positions and a primary or secondary alcohol under heating in the presence of a catalyst containing, as a main constituent, iron oxides.

The orthoalkylated aromatic amines are useful, as intermediates for producing pharmaceutical preparations, pesticides, dyes, resin stabilizers, rubber compounding ingredients and the like.

It has been known heretofore in the art, as a process for producing alkylated aromatic amines such as o-toluidine, 2,6-dimethylaniline and the like, that corresponding alkylated phenols such as o-cresol, 2,6-xylenol and the like are reacted with ammonia or amines under heating in the presence of a dehydration catalyst such as alumina, silica alumina or the like. However, there are problems in this reaction of the phenols having an alkyl group at the ortho-positions with ammonia or amines that the reaction rate is remarkably slow, as compared with the reaction of phenol with ammonia or amines and that the selectivity thereto and the yield thereof are low. Furthermore, since the decrease in the activity of the above-mentioned catalyst is remarkable, this method is difficult to use as a suitable method for industrially producing orthoalkylated aromatic amines.

It is also proposed in "Friedel Crafts and Related Reactions, Vol. 2, Part 1" by G. A. Olah, published by Interscience Publishers (1964) that aromatic amines such as aniline and the like are reacted with olefins or alcohols in the presence of a Friedel-Crafts type catalyst such as Lewis acids and the like. However, in the case where the amino group of aromatic amines is not substituted, N-alkylated aromatic amines such as N-monoalkyl aromatic amines, N,N-dialkyl aromatic amines or the like are produced as a main product and only a small amount of nucleal-alkylated aromatic amines are formed as by-products. In addition, a mixture of orthoalkylated aromatic amines and paraalkylated aromatic amines is formed as the nucleal-alkylated aromatic amines and the formation ratio of the orthoalkylated aromatic amines is low. This tendency is especially remarkable when an alcohol is used as an alkylating agent. Furthermore, in order to improve the problem of this method, it is proposed in Japanese Patent Publication No. 38-4569/63 that aromatic amines such as aniline and the like are reacted with olefins under the conditions of a high temperature and a high pressure in the presence of a Friedel-Crafts type catalyst. However, according to this proposed method, although the selectivity to the orthoalkylated aromatic amines somewhat increases, a still large amount of N-alkylated aromatic amines is formed as a by-product and the selective production of the orthoalkylated aromatic amines is difficult. Thus, the conventional processes for producing the orthoalkylated aromatic amines are by no means satisfactory as an industrial process.

Other processes for producing orthoalkylated aromatic amines are as follows. Proposed in U.S. Patent Specification No. 2814646 is a method in which aromatic amines such as aniline and the like are reacted with olefins under heating in the presence of a catalyst comprising aluminum anilide. Proposed in Japanese Patent Publication No. 47-24014/72 is a method in which aromatic amines such as aniline and the like are reacted with alkylaluminum halide, followed by the reaction of the resultant mixture with olefins. Also proposed in Japanese Patent Laid-Open Application No. 50-137934/75 is a method in which aromatic amines such as aniline and the like are reacted with lower olefins in the presence of a catalyst comprising aluminum anilide and halogenated hydrocarbons. Of these methods, the method disclosed in the above-mentioned U.S. Patent Specification No. 2814646 has a disadvantage that the reaction activity is low, although the selectivity to the orthoalkylated aromatic amines in the product is high. On the other hand, methods disclosed in the above-mentioned Japanese Patent Publication No. 47-240414/72 and Japanese Patent Laid-Open Application No. 50-137934/75 have advantages that the reaction activity is high and the selectivity to the orthoalkylated aromatic amines in the product is high. However, since the reactions of these methods should be carried out under a high temperature and high pressure, there is a disadvantage that a reaction apparatus suitable for use in the high temperature and high pressure conditions should be used and, since a substantial amount of the aluminum compound is used as a catalyst. A post treatment operation for removing the aluminum compound from the reaction mixture after the reaction is troublesome. Thus, these methods have many problems from a practical point of view. It should also be noted that, since olefins are used as an alkylating agent in these methods, orthomethylated aromatic amines such as o-toluidine and xylidine, and ortho-n-alkylated aromatic amines having an alkyl group of 3 or more carbon atoms cannot be produced.

Industrial & Engineering Chemistry, 43 (7), (1951) 1579—1583, discloses reacting aniline and, per mol thereof, 5 mol methanol, in the vapour phase at a temperature of 360°C, in the presence of various metal oxide catalysts, whereby N-methylaniline or N,N-dimethylaniline is produced. It is stated that silica gel and silica gel with iron oxide (ferric oxide on silica gel) were poor catalysts for alkylation. The section entitled "Effect of space velocity" states that there is an optimum velocity for tertiary amine formation while, at lower values, nuclear alkylation is favoured; the section entitled "Effect of temperature" indicates that the optimum temperature for maximum conversion to tertiary amine depends on the same factors as the space velocity, and that too high a temperature gives nuclear alkylation; and the section headed "Use of higher alcohols" suggests that there is a greater tendency towards nuclear alkylation when using, say, propyl and butyl alcohols.

2

# 0 073 277

According to the present invention, a process for producing an ortho-alkylated aromatic amine comprises reacting an aromatic amine having at least one ortho-hydrogen atom and a primary or secondary alcohol at an elevated temperature in the presence of a catalyst comprising a mixture of at least one iron oxide and at least one other metal oxide, in which the total weight of the metal oxides comprises at least 50% by weight of the at least one iron oxide.

The aromatic nucleus of the aromatic amine may be a benzene, naphthalene, anthracene or phenanthrene ring. The aromatic amine may be a primary, secondary or tertiary aromatic amine. The aromatic nucleus may carry one or more substituents, provided that at least one hydrogen atom is present at the ortho-position with respect to the amino or substituted amino group. Examples of typical substituents are alkyl, aryl, alkoxyl, aryloxyl, acyl, acyloxyl and hydroxyl groups, and halogen atoms.

Typical aromatic amines which can be used as a starting material in the present invention include aniline, o-toluidine, m-toluidine, p-toluidine, o-ethylaniline, m-ethylaniline, p-ethylaniline, o-isopropylaniline, m-isopropylaniline, p-isopropylaniline, 2,3-xylidine, 2,4-xylidine, 2,5-xylidine, 3,5-xylidine, 2,3-diethylaniline, 2,4-diethylaniline, 2,5-diethylaniline, 3,5-diethylaniline, 2,3-diisopropylaniline, 2,4-diisopropylaniline, 3,5-diisopropylaniline, N-methylaniline, N-ethylaniline, N-isopropylaniline, N,N-dimethylaniline, N,N-diethylaniline, N,N-diisopropylaniline, N-methyl-o-toluidine, N-methyl-2,3-xylidine, N-methyl-2,4-xylidine, N-methyl-2,5-xylidine, N-methyl-3,5-xylidine, N,N-dimethyl-o-toluidine, N,N-dimethyl-m-toluidine, N,N-dimethyl-p-toluidine, N,N-dimethyl-2,3-xylidine, N,N-dimethyl-2,4-xylidine, N,N-dimethyl-2,5-xylidine, N,N-dimethyl-3,5-xylidine, N-ethyl-o-ethylaniline, N-ethyl-m-ethylaniline, N-ethyl-p-ethylaniline, N-ethyl-2,3-diethylaniline, N-ethyl-2,4-diethylaniline, N-ethyl-2,5-diethylaniline, N-ethyl-3,5-diethylaniline, N,N-diethyl-o-ethylaniline, N,N-diethyl-m-ethylaniline, N,N-diethyl-p-ethyl-aniline, α-naphthylamine and β-naphthylamine. Of these aromatic amines, aniline and o-alkylanilines are preferred as the starting material in the present invention.

The alcohols used as a starting material in the present invention are primary or secondary alcohols, generally primary or secondary lower alcohols having 1 to 6 carbon atoms. Typical examples of the primary alcohols are methanol, ethanol, n-propanol, n-butyl alcohol, isobutyl alcohol, n-pentyl alcohol, isopentyl alcohol, n-hexyl alcohol, isohexyl alcohol and the like. Typical examples of the secondary alcohols are isopropanol, sec-butyl alcohol, sec-pentyl alcohol, sec-hexyl alcohol, cyclohexyl alcohol and the like.

Of the above-mentioned alcohols, the use of alcohols having 1 to 3 carbon atoms, especially methanol, ethanol or isopropanol is preferred. Although the amount of the alcohols to be reacted with the aromatic amines may be varied over a wide range, it is generally within the range of from 1 to 10 mol, more desirably 3 to 6 mol, based on 1 mol of the aromatic amine.

The catalyst may, if desired, be carried on a support or be bound using suitable binders. The catalytic metal oxides are such that the atomic ratio of the iron oxide or oxides to the other metal oxides, in terms of the metal element, is at least 50:50, preferably at least 70:30 and more preferably at least 90:10. The iron oxide or oxides can be in any form of $Fe_2O_3$, $Fe_3O_4$ or any mixture thereof.

One preferred group of oxides in the catalyst, other than the iron oxide or oxides, comprises the oxides of aluminium, gallium, silicon, germanium, tin, bismuth or magnesium. Another preferred group of oxides, other than the iron oxide or oxides, are those of the elements copper, titanium, zirconium, hafnium, vanadium, niobium, chromium, molybdenum, tungsten and rhenium.

The use of a mixture of metal oxides provides high selectivity to the ortho-alkylated aromatic amine and can effectively prevent decomposition of the alcohol during the course of the reaction. Especially when a small amount of metal oxides other than the iron oxide or oxides is used, e.g. in an atomic ratio of the metal element of the oxide to the iron in the iron oxide or oxides of from 0.5:100 to 10:100, as a tertiary component in an iron oxide-chromium oxide or iron oxide-magnesium oxide catalyst, the resultant 3-component catalyst, e.g. of the Fe-Ge-Cr or Fe-Si-Cr type, can have a desirably long catalyst life in addition to the advantages mentioned above.

The catalysts used in the process of the present invention can be prepared by, for example, the calcination of a mixture comprising iron and other metal compounds capable of forming iron oxides and oxides of the other metal, or the evaporation of an aqueous solution of such a mixture to dryness, followed by calcination.

In the case where the catalysts comprise iron oxides supported on carriers, a mixture as described above may be compounded with a carrier, and the compound then moulded and calcined. Alternatively, an aqueous solution of the mixture may be impregnated into a carrier and the resultant impregnated carrier calcined.

The process of the present invention may be carried out as a liquid or vapour phase reaction; a vapour phase reaction is preferred, in which case the temperature is generally 200 to 500°C, preferably 250 to 500°C. The liquid space velocity (LHSV) of the starting materials to be fed during the reaction is generally 0.05 to 10 $hr^{-1}$, preferably 0.1 to 2.0 $hr^{-1}$. Although the reaction can be generally carried out under a reduced pressure or under pressure, the reaction is preferably carried out under a pressure of 1 through 30 $kg/cm^2$-G. After the completion of the reaction, the unreacted alcohol is separated from the reaction mixture and, after recovering the unreacted starting aromatic amines, the desired orthoalkylated aromatic amines can be obtained by any conventional treatment, such as, distillation, crystallization, extraction or the like. The recovered unreacted alcohol and aromatic amines having at least one hydrogen atom at the

3

ortho-positions can be reused by circulating the same to the reaction system. The present reaction can be continuously or batchwise carried out.

Examples

The present invention is now illustrated by, but is by no means limited to, the following examples in which all percentages are expressed on a weight basis unless otherwise specified.

Example 1

202.0 g of ferric nitrate · 9 hydrates was dissolved in 2 liters of distilled water and, then, a 25% aqueous ammonia was gradually added to the resultant solution, whereby the pH of the solution became 7. The resultant precipitates were washed with water and filtrated. To the filtrated precipitates, 0.55 g of germanium dioxide was added and the resultant mixture was mixed for one hour by using an automatic mill. The mixture was dried at a temperature of 90°C for about 20 hours and, then, the mixture was calcined at a temperature of 450°C for 3 hours. Thus, a catalyst comprising iron oxides and germanium oxide was prepared.

20 ml of the crushed catalyst having a size of 6 through 10 meshes (Tyler No.) was packed into a reaction tube made of pyrex and having an inner diameter of 20 mm and, then, heated to a temperature of 370°C. After the temperature reached at the desired level, a liquid mixture having a mol ratio of aniline:methanol=1:5 was fed at a rate of 14 ml/hr, whereby the reaction was effected.

Example 2

The reaction was carried out in the same manner as in Example 1, except that a liquid mixture having a mol ratio of aniline:methanol:$H_2O$=1:5:2 was used as starting material.

Example 3

The reaction was carried out in the same manner as in Example 1, except that the methanol contained in the starting material was changed to ethanol.

Comparative Example 1

The reaction was carried out in the same manner as in Example 1, except that a commercially-available silica-alumina catalyst ($SiO_2$:$Al_2O_3$=85:15, weight ratio) was used in lieu of the iron oxide catalyst.

Comparative Example 2

The reaction was carried out in the same manner as in Example 1, except that $Fe_2O_3$ was used as a catalyst.

Example 4

The reaction was carried out in the same manner as in Example 1, except that $Fe_2O_3$-$SiO_2$ (97/3) was used as a catalyst.

Example 5

The reaction was carried out in the same manner as in Example 1, except that $Fe_2O_3$-$GeO_2$-$Cr_2O_3$ (96/3/1) was used as a catalyst.

Example 6

The reaction was carried out in the same manner as in Example 1, except that $Fe_2O_3$-$GeO_2$ (96/4) was used as a catalyst and a liquid mixture of aniline:isopropanol:$H_2O$ having a mol ratio of 1:10:2 was used as a starting material.

Example 7

The reaction was carried out in the same manner as in Example 1, except that $Fe_2O_3$-$SiO_2$-$Cr_2O_3$ (95/4/1) was used as a catalyst.

Example 8

The reaction was carried out in the same manner as in Example 1, except that $Fe_2O_3$-$GeO_2$ (70/30) was used as a catalyst.

Example 9

The reaction was carried out in the same manner as in Example 1, except that $Fe_2O_3$-$SiO_2$ (50/50) was used as a catalyst.

The results obtained in each of the Examples (and Comparative Examples) are tabulated below. The value for the "selectivity to desired product" is based on the alkyl group which is methyl in Examples 1, 2, 4, 5 and 7 and the two Comparative Examples, ethyl in Example 3 and isopropyl in Example 6.

4

| | Example 1 | Example 2 | Example 3 | Comparative Example 1 | Comparative Example 2 |
|---|---|---|---|---|---|
| Catalyst | $Fe_2O_3$-$GeO_2$ | $Fe_2O_3$-$GeO_2$ | $Fe_2O_3$-$GeO_2$ | $SiO_2$-$Al_2O_3$ | $Fe_2O_3$ |
| (Atomic ratio of metals) | (99/1) | (99/1) | (99/1) | (83/17) | (100/0) |
| Aniline conversion (%) | 84.1 | 71.2 | 46.2 | 92 | 32 |
| Selectivity (%) to desired product | | | | | |
| 2,6-Dialkylaniline | 44.8 | 51.6 | 37.0 | 0.6 | 4.5 |
| o-Alkylaniline | 42.2 | 40.2 | 49.2 | 1.3 | 69 |
| p-Alkylaniline | 0.4 | 1.2 | 1.2 | 18 | 0.1 |
| N-Alkylaniline | 8.5 | 5.2 | 8.4 | 72.6 | 25 |
| N,N-Dialkylaniline | 2.1 | 0.6 | 2.6 | 4.0 | 0.7 |
| o-Alkylation selectivity (%) | 87.0 | 91.8 | 86.2 | 1.9 | 73.5 |

| | Example 4 | Example 5 | Example 6 | Example 7 | Example 8 | Example 9 |
|---|---|---|---|---|---|---|
| Catalyst | $Fe_2O_3$-$SiO_2$ | $Fe_2O_3$-$GeO_2$-$Cr_2O_3$ | $Fe_2O_3$-$GeO_2$ | $Fe_2O_3$-$SiO_2$-$Cr_2O_3$ | $Fe_2O_3$-$GeO_2$ | $Fe_2O_3$-$SiO_2$ |
| (Atomic ratio of metals) | (97/3) | (96/3/1) | (96/4) | (95/4/1) | (70/30) | (50/50) |
| Aniline conversion (%) | 43 | 57 | 47.5 | 50.5 | 59 | 85 |
| Selectivity (%) to desired product | | | | | | |
| 2,6-Dialkylaniline | 13 | 43 | 35.1 | 39 | 34.1 | 32.1 |
| o-Alkylaniline | 74 | 54 | 60.2 | 50.1 | 45.3 | 38.2 |
| p-Alkylaniline | trace | 0.2 | trace | 0.2 | 0.2 | 1.7 |
| N-Alkylaniline | 8.5 | 0.6 | 3.1 | 8.4 | 20.4 | 17.5 |
| N,N-Dialkylaniline | 1.6 | 1.4 | trace | 0.5 | 6.4 | 7.4 |
| o-Alkylation selectivity (%) | 87 | 97 | 95.3 | 89.1 | 79.4 | 70.3 |

## 0 073 277

### Claims

1. A process for producing an ortho-alkylated aromatic amine, which comprises reacting an aromatic amine having at least one ortho-hydrogen atom and a primary or secondary alcohol at an elevated temperature in the presence of a catalyst, characterised in that the catalyst comprises a mixture of at least one iron oxide and at least one other metal oxide, and the total weight of the metal oxides comprises at least 50% by weight of the at least one iron oxide.

2. A process as claimed in claim 1, wherein the aromatic amine is aniline or *o*-methylaniline.

3. A process as claimed in claim 1 or claim 2, wherein the alcohol is methanol, ethanol or isopropanol.

4. A process as claimed in any preceding claim, wherein the reaction is carried out in the vapour phase.

5. A process as claimed in any preceding claim, wherein the reaction is carried out at a temperature of from 250 to 450°C.

6. A process as claimed in any preceding claim, wherein from 1 to 10 mols of the alcohol are used per mol of the aromatic amine.

7. A process as claimed in any preceding claim, wherein the catalyst comprises, in addition to the iron oxide or oxides, an oxide of aluminium, gallium, silicon, germanium, tin, bismuth or magnesium.

8. A process as claimed in any of claims 1 to 6, wherein the catalyst comprises, in addition to the iron oxide or oxides, an oxide of copper, titanium, zirconium, hafnium, vanadium, niobium, chromium, molybdenum, tungsten or rhenium.

### Patentansprüche

1. Verfahren zur Herstellung eines ortho-alkylierten aromatischen Amins, welches das Reagieren eines aromatischen Amins mit mindestens einem ortho-Wasserstoffatom und einem primären oder sekundären Alkohol bei einer erhöhten Temperatur in Gegenwart eines Katalysators umfaßt, dadurch gekennzeichnet, daß der Katalysator eine Mischung von mindestens einem Eisenoxyd und mindestens einem anderen Metalloxyd umfaßt und das Gesamtgewicht des Metalloxyds mindestens 50 Gew.% des mindestens einen Eisenoxyds umfaßt.

2. Verfahren nach Anspruch 1, dadurch gekennzeichnet, daß das aromatische Amin Anilin oder o-Methylanilin ist.

3. Verfahren nach Anspruch 1 oder 2, dadurch gekennzeichnet, daß der Alkohol Methanol, Ethanol oder Isopropanol ist.

4. Verfahren nach einem der vorstehenden Ansprüche, dadurch gekennzeichnet, daß die Reaktion in der Dampfphase ausgeführt wird.

5. Verfahren nach einem der vorstehenden Ansprüche, dadurch gekennzeichnet, daß die Reaktion bei einer Temperatur von 250 bis 450°C durchgeführt wird.

6. Verfahren nach einem der vorstehenden Ansprüche, dadurch gekennzeichnet, daß von 1 bis 10 Mole Alkohol pro Mol des aromatischen Amins verwendet werden.

7. Verfahren nach einem der vorstehenden Ansprüche, dadurch gekennzeichnet, daß der Katalysator zusätzlich zu dem Eisenoxyd oder -oxyden ein Oxyd von Aluminium, Gallium, Silizium, Germanium, Zinn, Wismut oder Magnesium umfaßt.

8. Verfahren nach einem der Ansprüche 1 bis 6, dadurch gekennzeichnet, daß der Katalysator zusätzlich zum Eisenoxyd oder -oxyden ein Oxyd von Kupfer, Titan, Zirkonium, Hafnium, Vanadium, Niob, Chrom, Molybden, Wolfram oder Rhenium umfaßt.

### Revendications

1. Un procédé pour la production d'une amine aromatique ortho-alcoylée qui comprend la réaction d'une amine aromatique ayant au moins un atome d'hydrogène en ortho et d'un alcool primaire ou secondaire à température élevée en présence d'un catalyseur, caractérisé en ce que le catalyseur comprend un mélange d'au moins un oxyde de fer et au moins un autre oxyde métallique, et le poids total des oxydes métalliques comprend au moins 50% en poids du ou des oxydes de fer.

2. Un procédé selon la revendication 1, dans lequel l'amine aromatique est l'aniline ou la o-méthylaniline.

3. Un procédé selon la revendication 1 ou 2, dans lequel l'alcool est le méthanol, l'éthanol ou l'isopropanol.

4. Un procédé selon l'une quelconque des revendications précédentes, dans lequel la réaction est effectuée en phase vapeur.

5. Un procédé selon l'une quelconque des revendications précédentes, dans lequel la réaction est effectuée à une température de 250 à 450°C.

6. Un procédé selon l'une quelconque des revendications précédentes, dans lequel on utilise 1 à 10 mol de l'alcool par mol de l'amine aromatique.

7. Un procédé selon l'une quelconque des revendications précédentes, dans lequel le catalyseur comprend, outre le ou les oxydes de fer, un oxyde d'aluminium, de gallium, de silicium, de germanium, d'étain, de bismuth ou de magnésium.

8. Un procédé selon l'une quelconque des revendications 1 à 6, dans lequel le catalyseur comprend, outre le ou les oxydes de fer, un oxyde de cuivre, de titane, de zirconium, de hafnium, de vanadium, de niobium, de chrome, de molybdène, de tungstène ou de rhénium.